Europäisches Patentamt

European Patent Office

Office européen des brevets

(11) Numéro de publication: **0 284 695**

**A1**

## (12) DEMANDE DE BREVET EUROPEEN

(21) Numéro de dépôt: **87400700.8**

(22) Date de dépôt: **30.03.87**

(51) Int. Cl.⁴: **A63B 71/00** , A61F 7/10

(43) Date de publication de la demande:
**05.10.88 Bulletin 88/40**

(84) Etats contractants désignés:
**AT BE CH DE ES FR GB GR IT LI LU NL SE**

(71) Demandeur: **CRISTOPIA S.A.**
**78 Chemin du Moulin de la Clue Quartier**
**Cayrègues**
**F-06140 Vence(FR)**

Demandeur: **Pisoni, Marc**
**76 rue de Turenne**
**F-75003 Paris(FR)**

Demandeur: **Favrelle, Christophe**
**Les Arènes F. 13 Avenue de Flotte**
**F-06400 Cannes(FR)**

(72) Inventeur: **Pisoni, Marc**
**76, rue de Turenne**
**F-75003 Paris(FR)**
Inventeur: **Favrelle, Christophe**
**Les Arènes F. 13, Avenue de Flotte**
**F-06400 Cannes(FR)**

(74) Mandataire: **Armengaud Alné, Alain**
**Cabinet ARMENGAUD AINE 3 Avenue**
**Bugeaud**
**F-75116 Paris(FR)**

(54) **Dispositif de rafraîchissement de parties du corps humain.**

(57) Dispositif de rafraîchissement destiné aux sportifs, caractérisé en ce qu'il comporte un bracelet (10, 24) souple, ergonomique, sur lequel est fixé un conteneur étanche (12, 22, 26) renfermant un matériau stockeur d'énergie frigorifique à forte chaleur latente de fusion-cristallisation, le conteneur et le bracelet étant recouverts d'un matériau absorbant.

EP 0 284 695 A1

**Fig.1**

## Dispositif de rafraichissement de parties du corps humain

Il est connu que certains matériaux à forte chaleur latente de fusion-cristallisation permettent de stocker une grande quantité d'énergie calorifique ou frigorifique dans un volume réduit. Par ailleurs, on sait qu'au dessus d'une température supérieure à 25°C environ, le rafraîchissement de certaines parties du corps humain apporte une sensation de bien être et qu'en outre, le rafraichissement peut calmer la douleur ou limiter les conséquences d'un choc, cette propriété étant utilisée, notamment, dans le domaine sportif.

Il existe à l'heure actuelle, notamment en vue d'applications thérapeutiques, des systèmes permettant d'appliquer localement du froid (poches à glace ou similaire) sur des parties du corps humain pour calmer la douleur. L'invention se propose d'apporter une autre application des matériaux stockeurs d'énergie frigorifique, destinée essentiellement aux sportifs et permettant d'une part d'éviter les inconvénients de la transpiration et d'autre part d'assurer leur rafraichissement.

En conséquence, cette invention a pour but un dispositif de rafraichissement pour sportifs caractérisé en ce qu'il comporte un bracelet souple ergonomique sur lequel est fixé par tout moyen approprié un conteneur étanche renfermant un matériau stockeur d'énergie frigorifique à forte chaleur latente de fusion-cristallisation, ledit conteneur et/ou ledit bracelet étant recouvert d'un matériau absorbant, par exemple du type tissu-éponge.

On comprend que le dispositif selon l'invention porté par le poignet du sportif permet d'obtenir un rafraichissement des veines du poignet et en outre de lutter contre les effets de la transpiration en utilisant le réflexe instinctif qu'ont les portifs de s'essuyer le front avec leur poignet.

Selon l'invention, le conteneur peut se présenter sous la forme d'une capsule amovible maintenue dans une échancrure du bracelet et comportant un têton servant de moyen de fermeture dudit bracelet. Les capsules peuvent être stockées dans un récipient hermétique, calorifugé, ce qui permet de procéder à un échange facile des capsules après épuisement de l'énergie frigorifique stockée.

Selon une variante de cette invention, ledit conteneur se présente sous la forme d'un sachet étanche renfermant le matériau frigorigène, ce sachet étant maintenu sur le bracelet par une bande de fixation du type "velcro".

D'autres caractéristiques et avantages de cette invention ressortiront de la description faite ci-après en référence au dessin annexé qui en illustre divers exemples de réalisation dépourvus de tout caractère limitatif. Sur le dessin :

Les figures 1 et 2 sont respectivement des vues en élévation et en plan d'un premier exemple de réalisation de l'invention ;

Les figures 3 et 4 sont respectivement des vues en plan et en élévation d'un premier exemple de réalisation d'un capsule contenant un matériau frigorigène utilisé dans le dispositif représenté aux figures 1 et 2 ;

Les figures 5 et 6 sont des vues similaires aux figures 3 et 4 illustrant un second exemple de réalisation d'une capsule ;

La figure 7 illustre une variante du dispositif selon l'invention ;

La figure 8 représente en élévation le conteneur renfermant le matériau à forte chaleur de fusion-cristallisation utilisé dans le dispositif de la figure 7 ;

La figure 9 est une vue en coupe du bracelet illustré par la figure 7 et

La figure 10 représente en coupe verticale un exemple de réalisation d'un système portable permettant le stockage des capsules selon les figures 3 à 6.

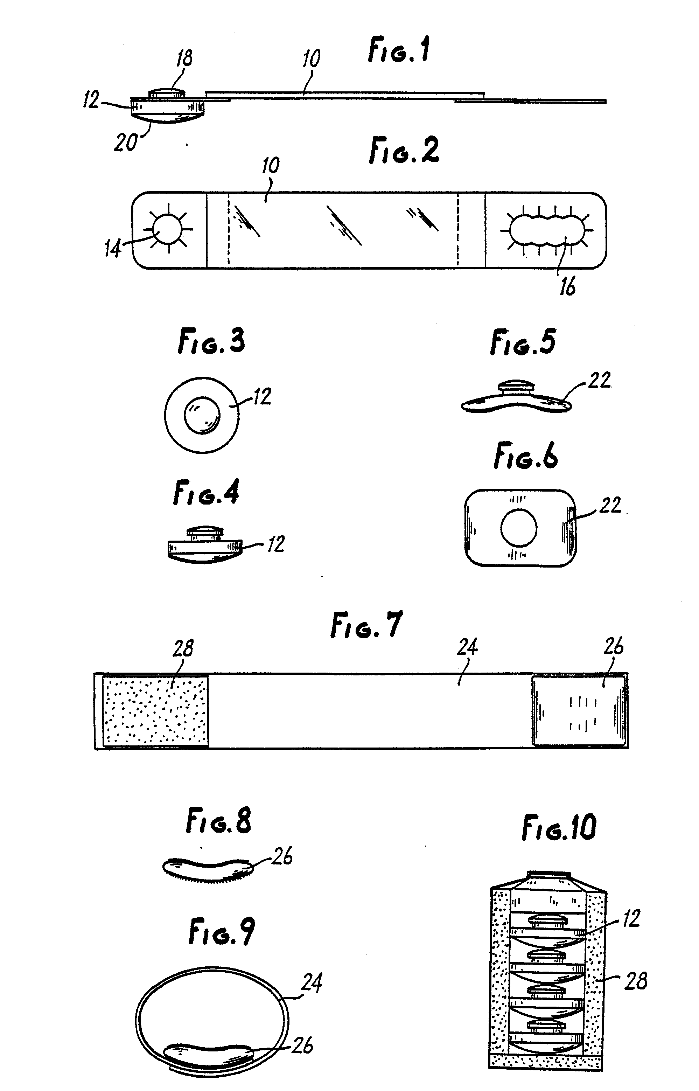

En se référant aux figures 1 à 6, on voit que selon ce premier exemple de réalisation de l'invention, le dispositif selon l'invention se présente sous la forme d'un bracelet 10 en matériau souple, agréable à porter, ergonomique, recouvert d'un matériau absorbant, par exemple du type tissu-éponge et sur lequel est fixée une capsule 12 étanche, de préférence en matière plastique, renfermant un matériau à forte chaleur latente de fusion-cristallisation. La capsule 12, munie d'un revêtement extérieur 20 en matériau absorbant, du type tissu-éponge par exemple, est encastrée dans le bracelet à l'aide d'un têton 18, en prise dans une échancrure 14, prévue à l'une des extrémités de la bande constituant le bracelet 10. L'autre extrémité de cette bande est munie d'une série de découpes 16 dans l'une desquelles vient s'engager le têton 18 qui sert ainsi de moyen de fermeture pour le bracelet.

Les figures 3 à 6 illustrent deux exemples de réalisation de capsules pouvant être utilisés avec le bracelet 10. La capsule 12 (figures 3 et 4) est de forme circulaire alors que la capsule 22 est rectangulaire et son profil (figure 5) est incurvé pour s'adapter à la forme du poignet de l'utilisateur.

On comprend que le bracelet 10 porté par un sportif permet non seulement de rafraîchir les veines du poignet de celui-ci mais également il peut être utilisé pour lutter contre la transpiration, le sportif ayant une tendance instinctive à s'éponger le front à l'aide de son poignet.

Sur les figures 7 à 9 on a représenté une variante de l'invention dans laquelle la bande 24 constituant le bracelet reçoit un sachet étanche 26, contenant le matériau frigorigène, fixé sur l'une des extrémités du bracelet à l'aide d'une bande du type "velcro" 28, le moyen de fermeture du bracelet étant une bande 26 du même type.

Les capsules 12, 22 et les sachets 26 peuvent être stockées avant usage, dans un récipient étanche et calorifique 28, (figure 10), ce recipient de petite dimension pouvant être porté par l'utilisateur, lequel peut facilement remplacer la capsule de son bracelet lorsque l'énergie calorigène qu'elle contient est épuisée.

Les capsules 12, 22 et les sachets tels que 26 son préalablement chargés en froid par passage en chambre froide puis introduits dans le récipient portatif 28, avant leur utilisation dans le bracelet selon l'invention.

Selon une autre variante de cette invention (non représentée) les capsules telles que 12, 22 et les sachets tels que 26 peuvent être fixés sur le bracelet en prévoyant des poches dans ce dernier, destinées au logement desdits sachets.

Il demeure bien entendu que cette invention n'est pas limitée aux exemples de réalisation décrits et représentés mais qu'elle en englobe toutes les variantes.

## Revendications

1-Dispositif de rafraichissement destiné aux sportifs caractérisé en ce qu'il comporte un bracelet (10, 24) souple, ergonomique, sur lequel est fixé un conteneur étanche (12, 22, 26) renfermant un matériau stockeur d'énergie frigorifique à forte chaleur latente de fusion-cristallisation, le conteneur et le bracelet étant recouverts d'un matériau absorbant.

2-Dispositif selon la revendication 1, caractérisé en ce que le bracelet est confectionné en matériau absorbant du type tissu-éponge.

3-Dispositif selon l'une des revendications précédentes caractérisé en ce que ledit conteneur est réalisé sous la forme d'une capsule étanche (12, 22), amovible, maintenue dans une échancrure (14) dudit bracelet et comportant un têton (18) en prise dans ladite échancrure et servant en outre de moyen de fermeture pour ledit bracelet.

4-Dispositif selon l'une des revendications 1 ou 2, caractérisé en ce que ledit conteneur se présente sous la forme d'un sachet (26) échanche, maintenu sur ledit bracelet (24) à l'aide d'une bande de fixation du type "velcro".

5-Dispositif selon l'une des revendications 1 ou 2, caractérisé en ce que le bracelet est muni d'une poche dans laquelle est logé ledit conteneur;

6-Dispositif selon l'une quelconque des revendications précédentes, caractérisé en ce que ledits conteneurs (12, 22, 26) sont chargés en froid en chambre froide, puis stockés avant leur utilisation, dans un récipient étanche calorifugé (28) portable, pouvant être porté par l'utilisateur.

0 284 695

**Fig.1**

**Fig.2**

**Fig.3**

**Fig.4**

**Fig.5**

**Fig.6**

**Fig.7**

**Fig.8**

**Fig.9**

**Fig.10**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 204 543 (HENDERSON)<br>* Colonne 1, lignes 53-58; colonne 2, ligne 40 - colonne 3, ligne 4; colonne 3, lignes 18-24,32-35; colonne 4, lignes 6-16; figures 2,3,7 * | 1,2,5 | A 63 B 71/00<br>A 61 F 7/10 |
| Y | | 3,6 | |
| Y | GB-A- 235 699 (SMITH et al.)<br>* Page 2, ligne 110 - page 3, ligne 18; figures 1-3 * | 3 | |
| Y | US-A-2 907 185 (ISHAM)<br>* Colonne 4, lignes 23-32 * | 6 | |
| X | WO-A-8 403 422 (BIGENI)<br>* Page 1, lignes 1-4; page 2, lignes 12-22; page 4, ligne 6 - page 5, ligne 10; page 5, ligne 17 - page 6, ligne 20; figure 1 * | 1,2 | **DOMAINES TECHNIQUES RECHERCHES (Int. Cl.4)**<br><br>A 63 B<br>A 61 F |
| Y | | 4 | |
| Y | WO-A-8 402 071 (HOSPIPHARM)<br>* Revendication 1 * | 4 | |

-/-

Le présent rapport de recherche a été établi pour toutes les revendications

| Lieu de la recherche | Date d'achèvement de la recherche | Examinateur |
|---|---|---|
| LA HAYE | 08-10-1987 | SCHOENLEBEN J.E.F. |

| Catégorie | Citation du document avec indication, en cas de besoin, des parties pertinentes | Revendication concernée | CLASSEMENT DE LA DEMANDE (Int. Cl.4) |
|---|---|---|---|
| **DOCUMENTS CONSIDERES COMME PERTINENTS** | | | Page  2 |
| A | DE-A-2 729 519  (DIECKMANN)<br>* Revendications 1,3,4,6,7;  figures 3,4 * | 3 | |
| | ----- | | |
| | | | **DOMAINES TECHNIQUES RECHERCHES (Int. Cl.4)** |

Le présent rapport de recherche a été établi pour toutes les revendications

| Lieu de la recherche | Date d'achèvement de la recherche | Examinateur |
|---|---|---|
| LA HAYE | 08-10-1987 | SCHOENLEBEN J.E.F. |

CATEGORIE DES DOCUMENTS CITES

X : particulièrement pertinent à lui seul
Y : particulièrement pertinent en combinaison avec un
　　autre document de la même catégorie
A : arrière-plan technologique
O : divulgation non-écrite
P : document intercalaire

T : théorie ou principe à la base de l'invention
E : document de brevet antérieur, mais publié à la
　　date de dépôt ou après cette date
D : cité dans la demande
L : cité pour d'autres raisons

&  : membre de la même famille, document correspondant

OEB Form 1503 03 82